# EUROPEAN PATENT SPECIFICATION

(11) **EP 0 644 929 B1**
(45) Date of publication and mention of the grant of the patent: **16.08.2000**
(21) Application number: 93915336.7
(22) Date of filing: 11.06.1993
(51) Int. Cl.: C12N 5/00, A61K 35/00, C12N 5/10, A61K 48/00, C12N 15/86, A61M 37/00

(54) **SYSTEM AND METHOD FOR TRANSPLANTATION OF CELLS**
SYSTEM UND VERFAHREN ZUR TRANSPLANTATION VON ZELLEN
SYSTEME ET PROCEDE POUR LA TRANSPLANTATION DE CELLULES

(30) Priority: 11.06.1992 US 897357
(43) Date of publication of application: 29.03.1995
(60) Divisional application: 99103453.9
(73) Proprietor: ERIKSSON, Elof, Wellesley Hills, Massachusetts 02181 (US)
(72) Inventor: ERIKSSON, Elof, Wellesley Hills, MA 02181 (US); VOGT, Peter, M., 44797 Bochum (DE)
(74) Representative: Ellis-Jones, Patrick George Armine
(86) International application number: PCT/US93/05665
(87) International publication number: WO 93/25660

(56) References cited:
- WO-A-87/00201
- WO-A-91/08793
- SURGICAL FORUM vol. 42, no. 0 , 1991 , CHICAGO IL, USA pages 670 - 673 E. ERIKSSON ET AL. 'In vivo cell culture accelerates reepitheliazation.'
- PROCEEDINGS OF THE NATIONAL ACADEMY OF SCIENCES OF THE USA vol. 86, no. 22 , November 1989 , WASHINGTON DC, USA pages 8803 - 8807 E. FENJVES ET AL. 'Systemic distribution of apolipoprotein E secreted by grafts of epidermal keratinocytes: Implications for epidermal function and gene therapy.'
- PNAS USA (1994) 91: 12188-12192
- SURGICAL FORUM (1995) 46: 702-705
- J. SURGICAL RES. (1992) 52: 50-58

## Description

### Background of the Invention

The invention relates to the use of exogenous genetic material in the manufacture of a medicament for use in a method of genetically engineering cells in vivo.

Keratinocytes are the principle cells which cover the surface of the body. They are capable of producing proteins, particularly keratin, which constitutes the main surface barrier of the body. For several different reasons, keratinocytes are attractive potential targets for gene transfer. Since they are located on the surface of the body, they are easily accessed both for gene manipulation and monitoring. If complications from gene transfer would occur, for instance, the development of local tumors or local infections, these could more easily be treated in the skin than elsewhere.

To date, genetic manipulation of keratinocytes has been done in one principal way. Skin has been harvested, the keratinocytes have been separated from the fibroblasts, and then the keratinocytes individually isolated and brought into suspension. These suspensions of keratinocytes have then been cultured to confluence using tissue culture techniques, as reported by Rheinwald, J.G., Green, H. Serial Cultivation of Human Epidermal Keratinocytes: The Formation of Keratinizing Colonies From Cells. Cell G, 331-343, 1975.

The new genetic material has been introduced into the keratinocyte while being grown *in vitro* using either a viral vector or plasmid, as reported by Morgan, J.R., Barrandon, Y., Green, H., Mulligan, R.C. Expression of an Exogenous Growth Hormone Glue by Transplantable Human Epidermal Cells. Science, Vol. 237, 1476-1479 (1987) and Tenmer, J., Lindahl, A., Green, H. Human Growth Hormone in the Blood of Athymic Mice Grafted With Cultures of Hormone-Secreting Human Keratinocytes. FASEB J., 4:3245-3250 (1990). The transfected cells are then usually resuspended and grown on selective media in order to increase the yield of transfection. Sheets of keratinocytes are then transplanted back to the mammal from which the keratinocytes were harvested.

Even though the *in vitro* yield has been acceptable, the *in vivo* yield has been unacceptably low, both short and long term. It has been very difficult to document any significant long term (more than thirty days) expression with these techniques, for example, as reported by Garlick, J.A., Katz, A.B., Fenvjes Esitaichman, L.B. Retrovirus Mediated Transduction of Cultured Epidermal Keratinocytes. J. Invest. Dermatol., 97:824-829, 1991.

The invention thus seeks to improve methods for transplanting genetically engineered cells into a patient, especially keratinocytes.

The present invention further seeks to provide a means for transplantation which is economical, practical, readily manufactured and can be customised to the patient with minimal effort and expense.

### Summary of the Invention

The present invention provides use of exogenous genetic material in the manufacture of a medicament for use in a method of genetically engineering cells *in vivo* in a patient by introducing the exogenous genetic material into the cells to be engineered, wherein a chamber formed of a flexible moisture and gas impermeable material is attached at a site on a patient where the cells are to be engineered and the chamber is open to the cells to be engineered and contains a fluid medium suitable for culturing cells.

The invention also provides use in the manufacture of a medicament of exogenous genetic material in combination with a chamber for culturing *in vivo* cells at a site on a patient, which chamber is formed of a flexible, moisture and gas impermeable material, and comprises an opening securable at the periphery to the skin of the patient,
wherein the combination is used in the manufacture of a medicament for use in a method for genetically engineering cells *in vivo* in a patient by introducing the exogenous genetic material into cells to be engineered wherein the chamber is attached at a site on a patient where the cells are to be engineered, the chamber containing a fluid medium suitable for culturing cells and being open to the cells to be engineered.

Gene transfer of genetic material with viral vectors, plasmids, or gene guns into keratinocytes, especially those including a high percentage of epidermal stem cells, in combination with the use of an "*in vivo*" culture chamber has been demonstrated to be particularly effective for culture keratinocytes.

By employing the wound chamber system, direct *in vivo* gene transfer can be done to exposed cells in an open wound. If these cells were not covered by the chamber, they would desiccate and die. The chamber also completely seals the wound from the outside, eliminating the spread of genetic material and vectors to places outside of the wound. At the same time, the chamber prevents the accidental introduction of undesired contamination, including viruses and other microorganisms and chemical contaminants into the wound.

The use of the wound chamber system for gene transfer also allows non-invasive assessment of the success of transfer by assaying for the presence of the expressed protein in wound fluid, in contrast to the prior art use of invasive techniques, such as biopsies, in order to achieve the same assessment of early expression.

Skin stem cells which are located in the hair follicles and in the basal layer of the skin are used to greatly enhance long term survival. Other cells, especially keratinocytes, can be used, alone or in combination with the stem cells.

A wide variety of proteins and materials can be expressed, either for secretion into the general blood and lymphatic system, or to alter the properties of the protein, for example, to not express proteins eliciting an immune response against the transplanted. cell.

### Brief Description of the Drawings

Figure 1 is a schematic of the exposed undersurface of a partial thickness skin flap used to expose hair follicles to obtain epidermal stem cells.
Figure 2 is a prospective view of a preferred embodiment of a chamber for culturing keratinocytes on a patient.
Figure 3 is a prospective view of a ported embodiment of a chamber for culturing keratinocytes.
Figure 4 is a schematic representation of a tattooing device for delivery of genetic material into cells.
Figure 5 is a cross-sectional view of a microneedle 10 shown in Figure 4.
Figure 6 is a photograph showing the insertion of genetic material into epidermis cells using a tattooing device.
Figure 7 is also a photograph showing the insertion of genetic material into epidermis cells using a tattooing device.
Figure 8 is a photograph of a histological slide showing keratinocytes in which genetic material has been inserted using the tattooing device.
Figure 9 is a photograph of a histological slide showing keratinocytes in which genetic material has been inserted using the tattooing device.
Figure 10 is a photograph of a histological slide showing keratinocytes in which genetic material has been inserted using the tattooing device.
Figure 11 is a graph showing expression of the gene encoding human growth hormone in keratinocytes, ng/ml versus time in days.
Figure 12a is a photograph of high frequency transfection with lacZ gene, β-Gal positive cells, of primary pig keratinocytes by retroviral gene transfer. Figure 12b is a photograph of expression of the lacZ gene in regenerated epithelium of a full thickness wound reconstituted by retrovirally transfected transplanted keratinocytes, β-Gal staining.
Figure 13 is a graph of protein leakage into wound fluid (mg/dl) over time (days), comparing the efflux of endogenous protein from full thickness wounds in six pigs for normal saline treatment (-squares-) versus keratinocyte suspensions (...triangles...) applied in treatment chambers to the wounds.
Figure 14a is a graph showing continuous production of human growth hormone (hGH) (ng/ml) over time (days) *in vitro* by primary porcine keratinocyte culture after retroviral gene transfer and is a mean of two experiments. Figure 14b is a graph showing human growth hormone (hGH) (ng/ml) over time (days) *in vivo* in wound fluid from full-thickness wounds produced by transplanted suspensions of porcine keratinocytes after retroviral gene transfer. After complete re-epithelialization (histologically, day 6) and formation of a multilayer epithelium, values return to baseline. Mean±SE (n=10)

### Detailed Description of the Invention

U.S. patent 5,152,757 describes a treatment system for wounds and other skin disorders including a chamber securable about the periphery of a wound, having portal means for introduction into and removal of treatment fluids from the chamber, treatment fluid, at least one treatment additive, control means for treatment variables, and monitoring means for monitoring wound conditions. Treatment fluid and additives are selected according to wound indications and include growth factors, antibiotics, anesthetics, enzymes and oxygen. Treatment variables such as temperature, colloid osmotic pressure, pH, ion concentration and oxygen content are controlled according to wound indications.

The chamber is secured about the periphery of a wound, a treatment fluid and at least one treatment additive introduced into the chamber, and the treatment variables controlled according to wound conditions. Examples of disorders that can be treated include wounds such as burns, pain, tumors, and infections. The chamber can also be used to culture cells onto wounds, where the dispersed cells are introduced into the chamber and allowed to settle and grow at a wound site.

The method described herein is based on two principal components. One is the targeting of epidermal stem cells for gene transfer. The second is the use of the wound chamber in order to create an *in vivo* tissue culture environment, which eliminates the need to culture cells *in vitro* when introducing the genetic material into the cells.

Epidermis keratinocytes are ideally suited for gene transfer and recombinant DNA expression in epidermal wound healing because of their key functions in mechanical stability and integrity and in secretion of biologically active substances, such as apolipoprotein A, which are detectable in the systemic blood circulation. It has been demonstrated that suspensions of genetically engineered keratinocytes can be transplanted onto open wounds, which eliminates the need to grow them into sheets. This makes the process of gene transfer in keratinocytes much easier and less time consuming.

### Cells.

Keratinocytes are separated from the dermis with dispase and then individually separated with trypsin and the fibroblasts removed. By targeting keratinocytes in the hair follicles, which provide a high number of stem cells, it has been found both that the yield of transfection is higher and that these cells are stably incorporated into the basal layer of the epidermis. The cells of the hair follicles serve as "stem cells of the epidermis". By using mechanical separation of the rest of the epidermis from this area these cells can either be removed using a dermatome technique or the genetic material can be directly introduced into these cells *in situ*.

Cells can be obtained as needed from other sites on the patient, including other tissues, for instance, bone marrow cells, muscle cells, and cells from endocrine organs.

### Introduction of Genetic Material

Genetic material can be introduced into the cells with plasmid, retroviral, or gene gun transfer using standard techniques. The genetic material would encode proteins such as growth factors, hormones, and other therapeutic proteins. These would speed the healing of wounds and correct certain deficiencies, such as parathyroid, growth hormone, and other hormone deficiencies, as well as deficiencies of certain clotting factors such as factor VIII. Cells can also be engineered to not express a protein, such as a protein involved in an immune response, for example, a human leukocyte antigen (HLA).

Keratinocytes are harvested and brought into suspension through exposure to enzymes, first to dispase and then trypsin, then grown to subconfluence in an incubator. Recombinant DNA is then introduced into the cell using a viral vector, plasmid or gene gun technique. After this the keratinocytes are suspended and injected into full thickness wounds which are covered by a wound chamber. These keratinocytes will not only survive, but will show continued presence of cells expressing the marker gene in the basal layer of the epidermis.

The keratinocytes and fibroblasts in the wound can be made to produce, for instance, growth factors, if the gene encoding the growth factor is introduced into the genome or cytoplasm of these cells. The expressed growth factor will then not only speed the healing of the wound, but also help to heal wounds that would not heal otherwise. The genetically engineered keratinocytes can therefore be used to deliver growth factors into the wound in a way that is determined by the wound environment itself.

This approach can be used to insert other genes of interest into keratinocytes in order to reinstitute functions defective in a variety of skin diseases. Also, the mode in which cytokines act can be changed in that a factor is expressed in a cell that normally does not produce that factor, thus changing a paracrine into an autocrine pathway. Instead of native cells, genetically engineered keratinocytes can be seeded and can secrete new protein into the wound during the healing process. In the same fashion, keratinocytes can be genetically engineered to repair inherent genetic defects, such as epidermolysis bullosa. These cells can then be seeded onto a superficial excisional wound to generate a new epidermis with the desired features. Problematic wounds require coverage of the defect and fast healing.

The following protocol is utilized for direct *in vivo* gene transfer in order to achieve systemic expression of the transferred gene.

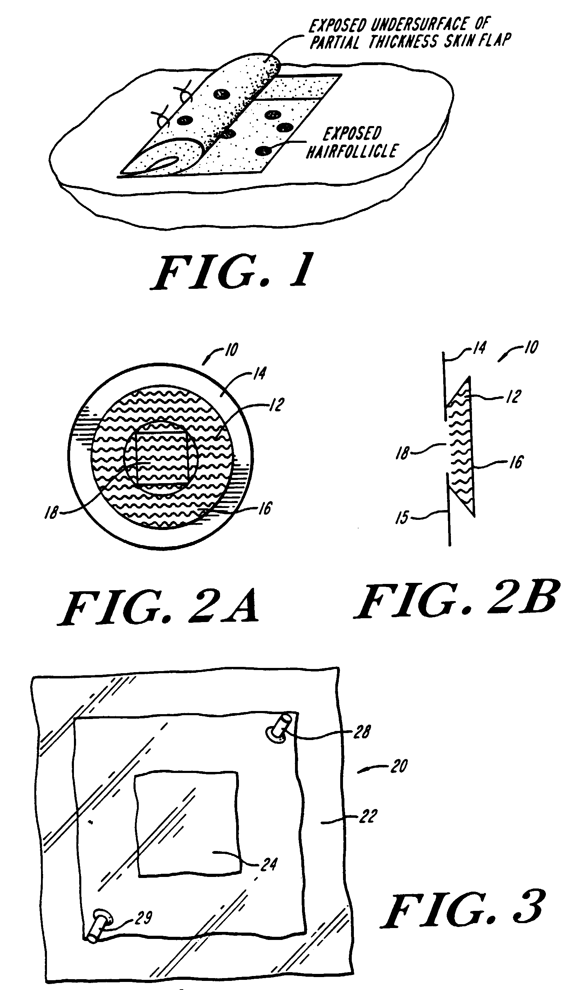

Stem cells located in the hair follicles deep to the epidermal dermal junction are exposed by creating a flap of epidermis where the deep portion of the flap contains the basal layer of the epidermis. The exposed wound surface contains the portion of the hair follicles with the stem cell keratinocytes. The genetic material is then introduced with a gene gun (ACCELL™, Dr. Dennis McCabe 1986) and then covered in a wound chamber. Alternatively, the wound with the exposed undersurface of the flap (see Figure 1) is exposed in the wound chamber and the gene in an appropriate vector for viral transfer or plasmid transfer is introduced into the chamber. Early expression of the gene is assayed in samples of this chamber fluid at 24 and 48 hours. After this the epidermal flap is sutured back in place and the wound is sealed with a dressing.

### The Chamber

In the preferred embodiment, cells are cultured in a system including a chamber, treatment fluid (which may be nutrient media, physiological saline, or some other compatible solution), treatment additives (such as antibiotics and buffering agents), means for controlling treatment variables and means for monitoring cell growth.

The wound chamber, which is made of vinyl or other flexible transparent material, such as polyurethane, has a bellows shape and an opening which corresponds to the size of the wound, either the chronic wound or a superficial wound created specifically for the purpose of gene transfer. The chamber contains a small amount of normal saline with antimicrobial agents. When plasmid or retroviral vectors are used, the chambers are simply attached over the wound and the vector is injected into the chamber and the chamber is then resealed. When the gene gun is used, the DNA is delivered into the cell and the chamber with normal saline and antibiotics is then attached to the perimeter of the wound. Wound fluid is sampled in order to assay expression of the secretable gene product (growth factor) at 24 and 48 hours. The wound is treated in the chamber until healed.

The chamber encloses a predetermined surface area about the culture site. The chamber provides protection for the wound from the surrounding non-sterile environment, control of treatment variables, containment for continuous fluid treatment, an effective delivery system for additives, direct monitoring of cell growth. Monitoring can be accomplished visually if the chamber is formed of a transparent material, or by extraction and analysis of fluid from the chamber. Fluid extracted from the system can be analyzed for factors which provide an indication of the status of healing, as well as the presence of undesirable components such as microorganisms, low oxygen, high carbon dioxide and adverse pH. The fluid may be tested for the number and type of bacteria and other microorganisms, the number and type of cells, the amount and type of proteins secreted by the patient and the cells within the chamber, and other factors such as drug levels, oxygen, carbon dioxide and pH.

The treatment system provides control over variables including temperature, specific ion concentration, colloid osmotic pressure, glucose concentration, amino acid content, fat concentration, oxygen concentration and carbon dioxide concentration and pH.

Portal means provide access for the introduction of treatment fluids and treatment additives into the chamber and extraction of fluid from the chamber. In some embodiments such as that shown in Figure 2, treatment fluid is introduced into the chamber by injection with a conventional hypodermic syringe through the wall of the chamber, preferably made of a flexible, self-repairing plastic. In other embodiments such as that shown in Figure 3, the chamber has an inlet and outlet port or separate inlet and outlet ports. Valve mechanisms are necessary where the apparatus is not to be connected to a treatment fluid reservoir and a drain or connected to a continuous perfusion system. The seals of the ports would be broken at an appropriate time for connection to other apparatus, such as a continuous perfusion system, at a hospital for example.

A preferred embodiment of the treatment system incorporates continuous perfusion of treatment fluid through inlet and outlet ports. A pump or gravity may be used to move the treatment fluid. The treatment fluid may be recirculated after filtering and other appropriate action (eg. heating or cooling). Alternately, fresh treatment fluid may be introduced and contaminated fluid disposed of. In an embodiment described in U.S. patent 5,152,757 the chamber contains a reservoir or more than one chamber, with the additional chamber serving as a source of fresh culture media, oxygen, and treatment additives.

Figure 2a and b show a preferred embodiment of a chamber 10, Figure 2a is a top view and Figure 2b is a side view. The chamber 10 has a bellows-type construction that prevents the majority of the chamber from contacting the wound service and in fact acts to push the chamber away from the wound. The chamber 10 is preferably constructed of clear vinyl or other sterilizable, flexible, heat-sealable, gas and moisture impermeable material. The bellows fold 12 allows for a large capacity for treatment fluid as well as ease of construction. For construction, an annular base 14 is heat or ultrasound welded to an annular bellows fold 12 piece and a circular top 16 welded to bellows fold 12 piece. Circular top 16 is a substantially transparent material. The remaining components are not necessarily transparent but may be. A transparent annular base may provide easy visual inspection for leakage. An opening 18 in annular base 14 is provided that corresponds in size to the wound or site for transplantation. The bottom side 15 of annular base 14 is provided with an adhesive tape or coating suitable for securing the chamber to skin.

As those skilled in the art will readily recognize, a removable sheet for protecting the adhesive and maintaining the sterility of the interior of the chamber is desirable. The chambers may be stored in a sterile pack for years. This chamber can take many shapes in order to fit wounds from the size of one square centimeter up to the size of a whole extremity. It is important that the adhesive surface be sufficient to secure the bandage to the skin surface to ensure a leak-proof seal.

As previously mentioned, treatment fluid and treatment additive introduction and subsequent extraction may be accomplished directly through the chamber walls by a needle and syringe. A self-repairing material to construct chamber 10 is contemplated. An alternative method would be to use inlet and outlet ports allowing the introduction and extraction of various substances into the chamber.

Figure 3 is a view of a chamber 20 for enclosing a predetermined surface area about a site on a patient where cells are to be cultured, where the chamber is open to the skin 24 and sealed at the edges 26 to the skin surface about the wound by means of an adhesive. The chamber 20 has a transparent section 22 for visual wound monitoring, a portal means 28, 29 for introduction of treatment fluid and treatment additives into the chamber and extraction of fluid from the protective chamber, and monitoring means (not shown) for analyzing extracted fluid for predetermined variables and wound conditions.

### Direct introduction of the genetic material into keratinocytes.

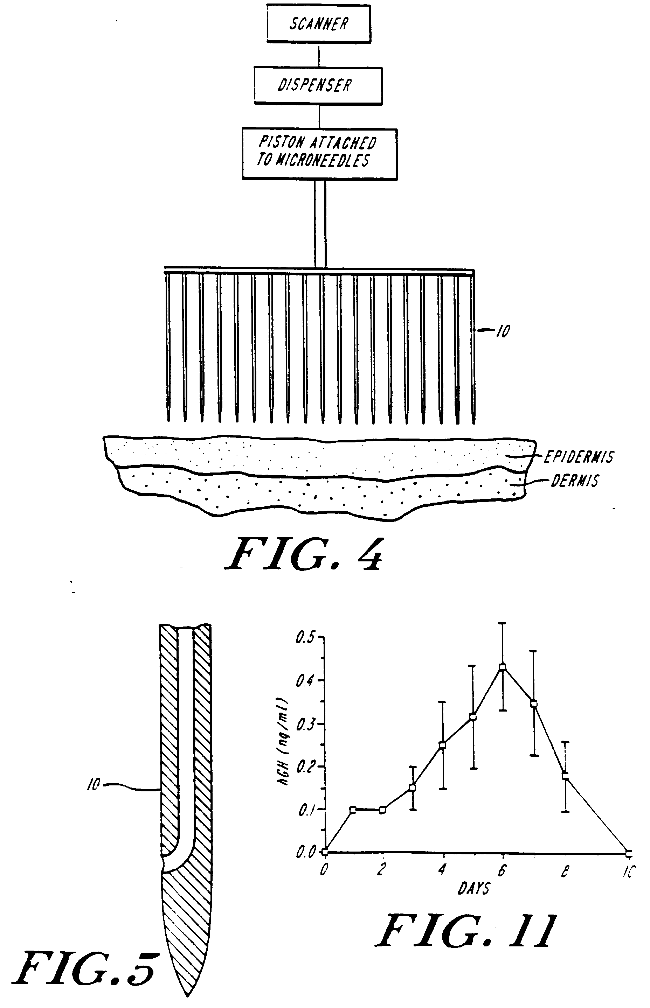

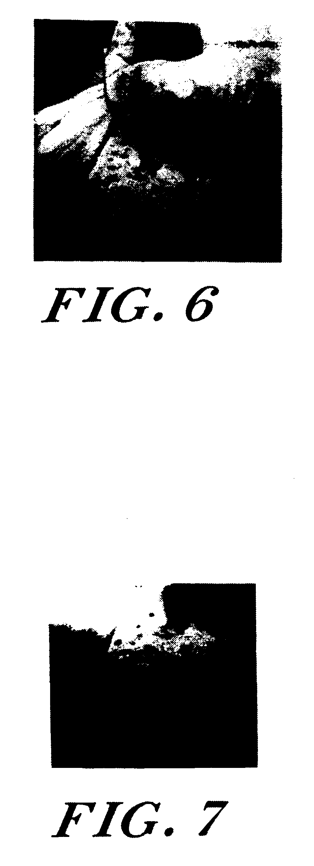

A crossbreeding of tattooing and microinjection can also be used to insert DNA material directly into cells. As shown in Figure 4, a plurality of microneedles 10 are mounted in a row on a handle. The microneedles 10 are pointed and hollow as shown in Figure 5. A solution containing genetic material, preferably DNA, is placed on the epidermal surface. The needles 10 are then moved as a group across the skin to "inject" and thereby deliver the genetic material through the surface of the skin to the underlying epidermis along several parallel lines. Microneedles such as tattooing needles are ideal for this purpose. Preferably, the needles are used to deliver microparticles, such as iron oxide microparticles having DNA material attached to them, as shown in Figures 6-10.

Alternatively, a row of microneedles is attached to a central dispenser containing a solution of microparticles to which the DNA material is attached and the solution is injected into skin cells, such as keratinocytes. In addition, this technique is useful for delivery of a suspension of DNA material alone, without attachment to microparticles.

This system, attached to a scanner, could systematically cover a specific area of skin or other tissue and deposit the DNA material very evenly into a large number of cells in the predefined area.

This method of introducing genetic material into keratinocytes may be superior to gene transduction using plasmids or retroviral vectors because the latter have an unacceptably low yield. Transfer of genetic material with accelerated particles, employing "gene guns", have a higher yield than plasmid or retroviral transduction, but have other disadvantages, such as a very loud blast and the risk of accidental discharge.

This combined technique of tattooing and microinjection of genetic material into cells with oscillating microneedles provides a practical, affordable and yet also a predictable method of cellular insertion of DNA material for systemic expression.

### Preparation of site for culturing cells.

The site for culturing cells is prepared by removing infected or burned skin, if necessary, or by creating an appropriate wound for transplantation of engineered cells. The skin adjacent to the wound is then cleaned so that there will be good adhesion between the chamber and the skin. The open portion of the chamber is then placed over the site, with the adhesive edges securing the chamber to the skin, then an appropriate culture medium and cells are introduced into the sealed chamber. The treatment fluid may be introduced and then extracted in favor of fresh culture media in a continuous or batch process. Selected treatment additives may be introduced into the chamber continuously or at a predetermined time or at periodic intervals. Appropriate control of treatment variables is also effected. Monitoring is accomplished by examination of the patient and visual examination of the fluid within the chamber and the wound itself. In addition, samples of fluid are extracted from the chamber for analysis and diagnosis. The chamber is removed once sufficient healing of the wound has occurred.

For example, to determine whether or not the wound is healed, the protein content of the extracted fluid is analyzed. When the protein content of the extracted fluid decreases to the level present in chambers containing fluid that are placed over normal skin, the wound is healed. Methods for determining protein content is well known in the art and are inexpensive and fast. The types of protein and the relative amounts of the types of protein can also be determined to further evaluate healing and expression of exogenous genetic material.

### Control of Treatment or Culture variables.

Treatment of each patient is specific for the conditions within the chamber. Control over treatment variables can include continuous cooling to 34°C for the first 24 hours. Monitoring can include analyzing extracted fluid for protein and microorganisms, with samples extracted every 24 hours. For example, when the number of microorganisms is less than 10 to the 4th per milliliter or per cc, infection has been resolved. Protein levels checked every day should be less than 24 mg/dl/cm².

As noted above, there are a number of treatment variables which may be controlled by the system. One such treatment variable which may be controlled is temperature. It has been found that heating the wound from a temperature of approximately 27°C (a common temperature of a lower extremity wound) to 37°C accelerates wound healing. Experimental data has shown that at a wound temperature of approximately 37°C, the rate of wound healing is more than twice as fast as at a temperature of 27°C. The temperature of the wound area can be achieved by heating the treatment fluid. Cooling has also been proven beneficial in the case of acute burn and other traumatic wounds. Cooling reduces pain, swelling and destruction of tissue. In general terms, acute wounds benefit from cooling during the first hours after occurrence of the wound and later, wounds benefit from a temperature of approximately 37°C. Cooling can similarly be effected by cooling the treatment fluid.

Other treatment variables may also be optimized. For example, ion concentrations should be kept close to extracellular ion levels. Glucose, amino acid and fat concentrations should be kept close to the concentrations present in plasma or corresponding to a skin tissue culture medium. Oxygen and carbon dioxide concentrations should also be maintained at their normal tissue levels. Oxygen is an important treatment additive, and is essential for cell growth.

### Treatment additives and Culture Media.

Normal or physiological buffered saline is the basic culture media. Buffering agents, anesthetics such as lidocaine, antibiotics such as penicillin or streptomycin, chemotherapeutic agents, and growth factors including epidermal growth factor (EGF), platelet-derived growth factor (PDGF), insulin-like growth factor (IGF), basic fibroblast growth factor (bFGF), and cholera toxin (CT) can be added to the culture/treatment media. Tissue culture mediums and fluids which increase osmotic pressure and oxygen accessibility may also be introduced to the chamber as treatment additives.

Selection of treatment additives is wound specific. For example, if an infection has been diagnosed, antibiotics are added in the amount of one single parenteral dose per 1,000 cc of fluid. Furthermore, a treatment additive of gentamicin, tobramycin or carbenicillin is appropriate for a wound infection with *Pseudomonas*, detected by analyzing extracted fluid. When hypoxia has been diagnosed, the liquid is passed through an oxygenating chamber before entering the chamber. If a tumor has been diagnosed, chemotherapy is given in an amount of one single parenteral dose per 1,000 cc of fluid. In situations involving a wound containing necrotic tissue and debris, proteolytic enzyme is added to the liquid. Immune modulators are added to the treatment fluid if an inflammatory reaction is exhibited. Epidermal growth factor is added in a concentration of 10 nanograms per cc when required.

The present invention will be further understood be reference to the following non-limiting examples.

### Example 1: Transfection of keratinocytes and expression of human growth hormone in culture and following transplantation into partial thickness wounds.

In the first series of experiments, keratinocytes were harvested, plated in culture flasks, and transfected with a viral vector technique, using the method of Wilson, J.M, Biriuyi, L.U., Salomon, R.U., et al. Transplantation of Vascular Grafts Lined With Genetically Modified Endothelial Cells. Science, 244:1344-1346 (1980), or a plasmid transfer technique, as described by Felgner, P.L., Galik, T.R., Holmer, et al. Lipofection: An Efficient, Lipid Mediated DNA-Transfection Procedures. Proc. Natl. Acad. Sci., 84:7413-7417 (1987), the teachings of which are incorporated herein.

Pigs are the standard animal model for extrapolation to human skin because porcine skin resembles human skin in several respects, including turnover time.

Specifically, keratinocytes were harvested from two female Yorkshire pigs by partial thickness skin excision, dispase separation of epidermis (0.25% Dispase, 2-3 h at 37°C), trypsinization (0.1% Trypsin, 0.02% EDTA, 30 min at 37°C) and mincing to a single cell suspension. They were grown to subconfluence (60-70%) and incubated with a replication defective murine Moloney leukemia retroviral vector. The DNA sequence for hGH was inserted into the viral genome and was under the promoter control of the viral long-terminal repeats (LTR's). The infected keratinocytes were trypsinized, resuspended in saline containing penicillin (100 U/ml) and streptomycin (100 µg/ml). They were transplanted to full thickness wounds created on the dorsum (n=10) in an autologous fashion. Full thickness wounds seeded with unmodified keratinocytes (n=10) and saline (n=10) served as controls. All wounds were covered with sterile vinyl chambers for maintenance of a liquid wound environment throughout the study. The presence of hGH in the culture medium or the wound fluid was determined by radioimmunoassay.

The same methodology was used with the lacZ gene, an intracellular marker gene.

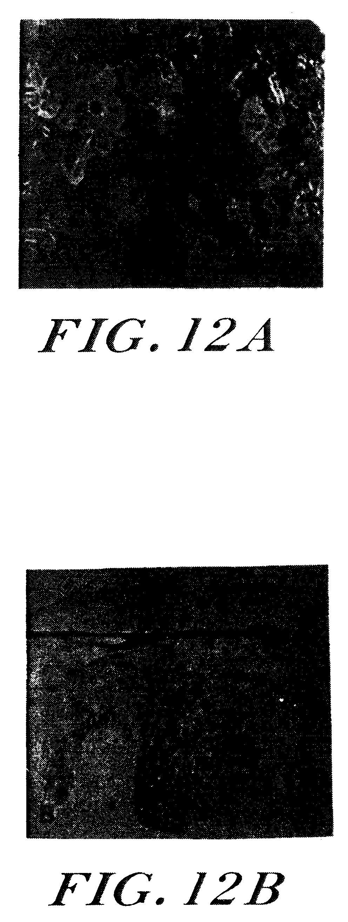

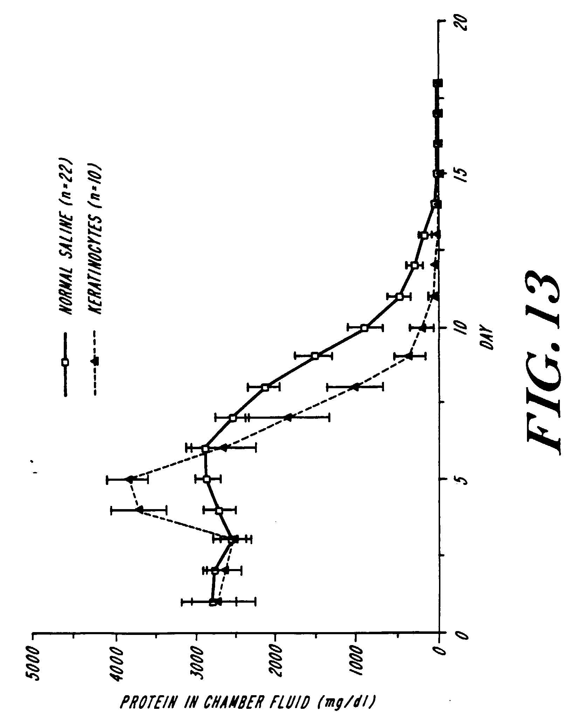

Significant expression of both genes in this system was obtained, as shown by Figures 11 and 12a and b. Histologic sections of the skin showed that the keratinocytes that had the lacZ marker gene were not stably incorporated into the basal layer, but migrated to the surface and were lost into the stratum corneum. When analyzing the reasons behind this, it was concluded that the dispase separation of dermis from epidermis did not allow for harvesting of a sufficient amount of stem cells. An additional reason was that the repeated trauma of harvesting the cells, exposure to tissue culture medium with relatively high calcium, and the cellular injury from the virus or the plasmid medium, caused a near terminal differentiation of the keratinocytes. Accordingly, results were greatly improved by using a higher percentage of epidermal stem cells obtained from the hair follicles, and decreasing the exposure to calcium and viral or bacterial medium.

*In vitro* (culture medium) hGH was detected on day 4 after retroviral transfection with a mean daily production of 2.0 ng/ml. *In vivo* (wound fluid) hGH production was first detected 24 hours after transplantation (0.1 ng/ml) peaked at 132 h (0.42 ng/ml) and returned to baseline by day 10. Healing times, assessed by the protein efflux assay in wound fluid, wounds receiving hGH-keratinocytes (12.1 ± 1.0 days) were no different than those of wound receiving non-infected keratinocytes but significantly shorter (p < 0.005) than non-transplanted control wounds (14.7 ± 0.6 days). Histologically transplanted keratinocytes established a multilayer epithelium as early as day 6 (saline treated controls by day 11).

The results demonstrate that autologous keratinocytes carrying the hGH gene will continue to express the hGH gene when transplanted to full thickness wounds. The produced hGH does not influence healing times. Compared to the *in vitro* keratinocyte cultures hGH production *in vivo* is regulated in a time dependent fashion. This may be either due to a regulation of hGH gene expression by keratinocytes during epithelialization, loss of hGH producing keratinocytes due to terminal differentiation, or antibody reaction to the foreign protein, as discussed by Selden, et al., "Human Growth Hormone as a Reporter Gene in Regulation Studies Employing Transient Gene Expression" Mol. Cell Biol. 6(9):3173-3179 (1986). Since genetically engineered keratinocytes not only improve re-epithelialization of full thickness wounds but also release a recombinant DNA product during healing they can be used to deliver and express therapeutic peptides, like growth factors, during wound healing. By using a topical chamber system, direct application of genetically engineered cells and monitoring of the gene product can easily be facilitated.

### Example 2: In Vivo Culture of Genetically Modified Keratinocytes.

Unmodified and retrovirus-transduced keratinocytes expressing the β-galactosidase gene and human growth hormone gene (hGH) were transplanted into enclosed skin wounds of Yorkshire pigs. Analyses of sequential skin samples obtained by biopsy during the four weeks after transplantation revealed survival of the transplanted cells and expression of β-galactosidase. Transfected keratinocytes were first documented in the deep portions of the wounds and then in the basal layer of regenerated epidermis. These cells contributed to the normal skin architecture and barrier function and transferred the new gene product to terminally differentiating cells of the stratum spinosum. When keratinocytes transduced with the hGH gene were transplanted into wounds, hGH was detected for up to 10 days in wound fluid. In contrast, hGH production *in vitro* was continuously detectable for 47 days. The pattern of expression demonstrates that transduced keratinocytes reconstitute the new epidermis and undergo terminal differentiation and could be used for the introduction and expression of Therapeutic proteins in skin disease to promote skin replacement and healing.

Porcine wounds (partial and full thickness burns, incisions and excisions) and human wounds (chronic full thickness) were individually enclosed in vinyl chambers containing placebo fluid with additives. Unless otherwise specified, the placebo fluid was unbuffered 0.9% NaCl with 100 IU of penicillin/ml and 100 µg of streptomycin/ml. Various media for the culture of keratinocytes were also used. Maintenance of the chamber system took place twice daily, daily, every other day, or continually. The wounds were treated in the chambers until healed. The wounds were assessed macroscopically, biochemically (by analysis of the chamber fluid) and microscopically. Both wound fluid and human growth hormone (hGH) in vitro culture fluid were assessed in the Allegro™ Radio-Immuno-Assay (Nichols Institute Diagnostics, San Juan Capistrano, CA)

The β-galactosidase gene of *Escherichia coli* (lacZ) was inserted into porcine keratinocytes through transduction with a murine Moloney leukemia vector in which the β-galactosidase gene is under control of the retrovirus long terminal repeat, as described by Danos et al., *Proc. Natl. Acad. Sci*. 85:6560-6464 (1988). Suspensions of native or retrovirally transduced keratinocytes were transplanted into surgically-created full-thickness skin wounds in 13 female yorkshire pigs with a vinyl chamber as a temporal *in vivo* incubator. Porcine skin resembles human skin in several respects (e.g. turnover time) and therefore was the most appropriate model for this study. Domestic female Yorkshire pigs (3-4 months old; 40-45 kg) were used. Primary keratinocyte cultures were established from partial thickness skin grafts (0.012" thick) taken from the dorsal neck region of the pigs under general inhalational anesthesia. Sections were incubated in serum free medium containing 0.25% Dispase™ at 37°C for 2 to 3 hours to separate the epidermis from the dermis. A single cell suspension of keratinocytes was obtained by incubation in 0.1% trypsin and 0.02% EDTA (37°C for 30 minutes) and careful mechanical disruption. The solution was filtered through a 100 µm mesh and resuspended in medium. Cells were then seeded into culture flasks at a density of 0.12 x 10⁶ cells/cm² and grown in Weymouth medium containing 20% fetal bovine serum.

At 60-80% confluence keratinocyte cultures assigned for gene transfer were transfected with an amphotropic helper-free murine leukemia virus (ψ-CRIP) containing the β-galactosidase gene (lacZ). Subconfluent cell cultures were incubated on three consecutive days with the retrovirus-containing medium. Unmodified keratinocytes were grown to confluence without manipulation.

A total of 170 full thickness wounds were created on the backs of 13 pigs, with the panniculus carnosus muscle left intact. After careful hemostasis a liquid-tight vinyl chamber serving as an *in vivo* cell culture device was applied to each wound. The chamber (P.A. Medical Corp., Columbia, TN) consists of a flexible transparent vinyl top bonded to an adhesive base. The base has a central opening compatible with the margin of the created wound. Chambers were filled with 1.2 mL of normal saline containing 100 µg streptomycin and 100 IU penicillin/ml (n-67), transduced skin keratinocytes (lacZ, n=22 hGH, n=10) or nontransfected keratinocytes (controls, n=71). Starting on day 2 (to give the cells time to attach to the surface) the saline chambers were exchanged and refilled on a daily basis.

The effects of transplantation of keratinocytes on re-epithelialization were studied macroscopically, histologically, and by measurement of endogenous proteins in chamber fluid (as a noninvasively evaluable parameter of epithelial barrier function). Fluid collected from individual wounds was centrifuged at 2,000 x g for 10 minutes and then passed through a filter (pore size 0.45 mm, Millipore, Corp., Bedford, MA). Endogenous proteins as a noninvasive marker of restoration of epithelial barrier function was measured with a turbidimetric assay (STANBIO CSF, Stanbio Laboratory Inc., San Antonio, TX) as described previously by Breuing et al., J. Srug. Res. 52:50-58 (1992).

Chamber-treated wounds developed no tissue necrosis while wounds covered with conventional dressings were significantly necrotic. This difference was visible macroscopically. After 4 days a fibrinous clot had developed in chamber treated wounds of all groups. From day 6 on, wounds into which either unmodified or retrovirus-transduced keratinocytes had been transplanted were covered with a thin epithelium; this development was not seen in saline controls until day 12 as shown in Figure 13. Wound contraction did not differ in the three groups.

The macroscopic appearance of porcine full thickness wounds on days 3, 6, and 9 after wounding were examined. At confluence, keratinocytes were released from flasks with 0.01% trypsin and resuspended in normal unbuffered saline (0.9%) containing 100 U penicillin and 100 µg streptomycin/ml, at a concentration of 3 x 10⁶ cells/ml. A 1.2 ml volume of this suspension was injected into full-thickness wounds (15 x 15 mm, 9 mm deep) created on the pig dorsum. Keratinocytes resuspended in saline were found to be more than 90% viable, as confirmed by trypan blue uptake and reculturing of the content of individual syringes in culture flasks. Means and standard errors were calculated for the groups. Statistical significance between groups was analyzed with the nonparametric Mann-Whitney U-test. A logistic regression analysis of the reestablishment of the neoepidermis, as evaluated histologically, was performed.

Wounds into which transplanted keratinocytes were transplanted developed an early epithelium on day 6, while saline treated controls still contained a clot that was not re-epithelialized clot. The rate of wound contraction was not affected. Therefore, the epithelial barrier was established significantly earlier after transplantation of suspensions of autologous keratinocytes than after treatment with saline only (mean ±SD 12.66±16 days versus 14.7±.7 days; p < 0.05).

This difference was confined histologically by documentation of complete epithelialization on day 12 after transplantation but only on day 16 after treatment with saline. In wounds with transplanted keratinocytes, the following characteristic pattern was observed. Retrovirus transduced, lacZ-expressing keratinocytes first appeared in clusters on the bottom of the wounds on day 4 and then migrated upward with the newly developing matrix, as seen on day 6. By day 8 they were present in all layers of epithelium. Therefore, clusters of these cells were first seen on day 4 on the deep wound surface and later in the developing matrix. These clusters were evident until a new epithelium developed and disappeared thereafter.

*In vitro* staining of wounds with transplanted lacZ-positive keratinocytes revealed β-galactosidase positive cells within clusters of colonies on the bottom of the wound, closely related to the subcutaneous tissue on day 4. On day 6, the cells appeared in the center of the newly-developing connective tissue. Serial sections stained with hematoxylin and eosin and with Masson's trichrome showed that these nests were surrounded by dense collagen bundles. By day 8 lacZ-expressing keratinocytes were distributed in all cell layers of the newly regenerated epithelium including the stratum germinativum. Wounds biopsied at 27 days showed lacZ expression only in the upper portion of the stratum granulosum and not in the stratum basale or the stratum spinosum. Cross-sectional biopsy samples including margins of unwounded skin were taken from the wounds from day 4 to day 27. Half were fixed in 10% formalin, embedded in paraffin and stained with hematoxylin/eosin, or trichrome. The other half were snap-frozen in liquid nitrogen, sectioned, and stained for the detection of β-galactosidase-activity *in situ* with the substrate 5-bromo-4-chloro-3-inolyl β-D-galactoside (X-Gal chromogen), which forms a blue precipitate in transduced cells. By day 27, no additional nests of keratinocytes were detected in the subepidermal tissue.

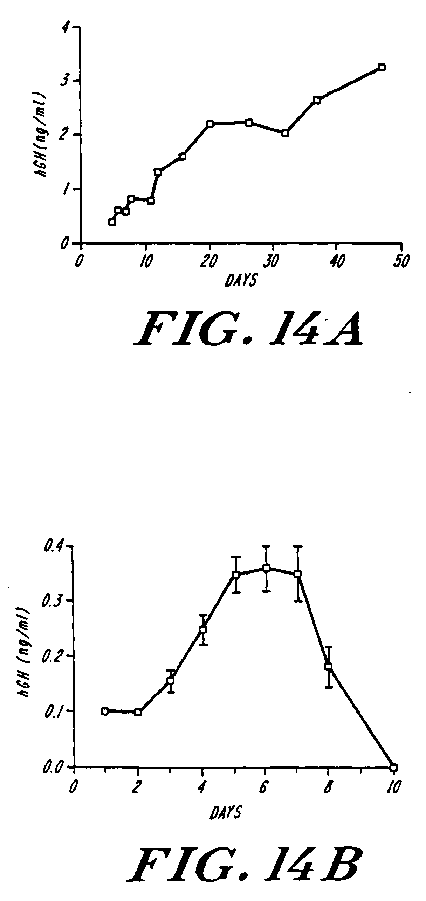

The histological appearance of the epithelium after transplantation of either transduced or unmodified keratinocytes was similar and was characteristic of a cornified multilayer epithelium. Horizontal sections through the epidermis showed evidence of an even distribution of transduced cells (280/mm²). Keratinocytes transfected with both *lacZ* and hGH (n=34 wounds) transplanted into the wounds produced hGH in a temporary fashion, with concentrations peaking at day 6 and declining to 0 until day 10 as shown in Figure 14b.

This experiment demonstrates that suspensions of keratinocytes can be stably transduced with a gene and that these cells continue to express the gene after transplantation into full thickness skin wounds in pigs. In a cutaneous chamber system, the cells were successfully transplanted, survived, and proceeded from cluster formation to reconstitution of new epithelium in a predictable fashion. The epidermis stratified and formed a functionally intact epithelial barrier. The wet environment of this closed-chamber system creates a favorable environment for the transplantation of cells. It includes all the necessary components such as serum electrolytes at physiologic osmolality and pH and a variety of growth factors (e.g. basic fibroblast growth factor epidermal growth factor) that exhibit mitogenic activity for keratinocytes.

The decreasing expression of lacZ may be explained by an early and rapid terminal differentiation of the transduced keratinocytes, which is likely when the specific pattern of lacZ expression is taken into account. The high calcium concentration in fluid as well as the presence of other mediators and the interactions of these mediators with cells in the wound may induce terminal keratinocytes differentiation. The morphologic and functional formation *in vivo* of an epithelial barrier of lacZ-expressing keratinocytes indicates that the cells have stratified and terminally differentiated. This conclusion is supported by the finding of lacZ-expressing keratinocytes only in the uppermost stratified layers of the epithelium after 27 days. Therefore, this experiment shows that suspensions of retrovirus transduced keratinocytes form a multilayered epithelium *in vivo* after autologous transplantation into full thickness wounds. Moreover, these cells provide function in a manner similar to unmodified keratinocytes in that they stratify and form an epithelial barrier to water and protein loss. Thus it is possible to regenerate a new epithelium with physiological properties through the use of genetically manipulated keratinocytes.

### Example 3: Introduction of DNA into keratinocytes and fibroblasts using "tattooing".

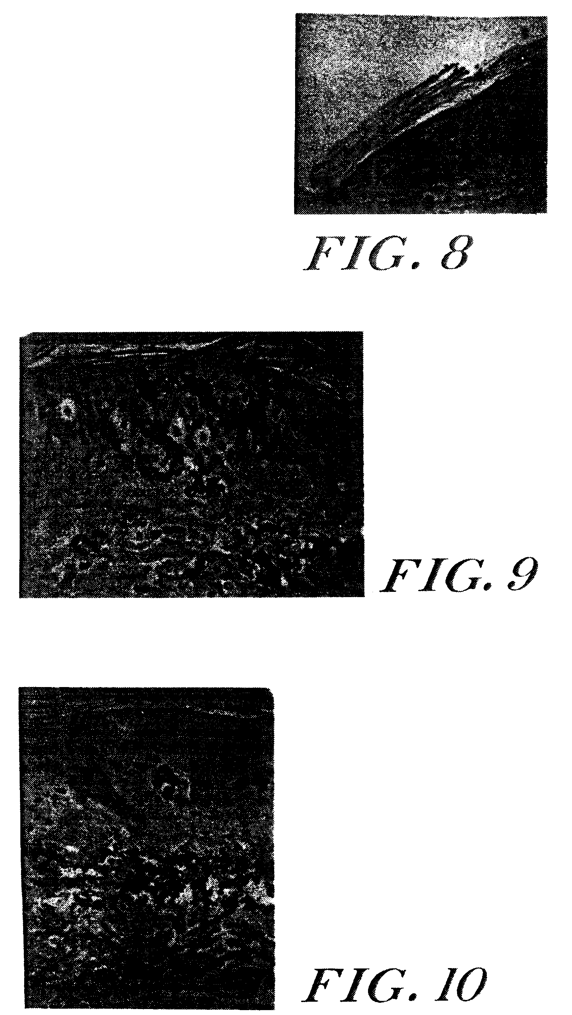

Iron oxide particles ranging in size from 0.05 microns to 1 micron in diameter were mixed with DNA plasmids in Tris-EDTA buffer. Drops of this material were placed on intact human skin. A tattooing device, containing microneedles, was placed on the material on the skin to insert, or inject, the DNA into superficial keratinocytes as shown in Figure 6, as well as stem cell keratinocytes in the deep epidermis, as shown in Figure 7, or denial fibroblasts, as shown in Figure 8.

## Claims

1. Use of exogenous genetic material in the manufacture of a medicament for use in a method of genetically engineering cells *in vivo* in a patient by introducing the exogenous genetic material into the cells to be engineered, wherein a chamber formed of a flexible moisture and gas impermeable material is attached at a site on a patient where the cells are to be engineered and the chamber is open to the cells to be engineered and contains a fluid medium suitable for culturing cells.

2. Use according to claim 1 wherein the cells are keratinocytes.

3. Use according to 1 or 2 wherein the genetic material is in the form of a viral vector or plasmid.

4. Use according to any one of claims 1 to 3 wherein the cells are engineered to express exogenous genetic material.

5. Use according to claim 4 wherein the cells are engineered to express proteins selected from hormones, immunomodulators, clotting factors, and growth factors.

6. Use according to any one of claims 1 to 3 wherein the cells are engineered to not express proteins eliciting an immune reaction against the cells.

7. Use according to claim 6 wherein the cells do not express HLA antigens.

8. Use according to any one of the preceding claims wherein culture conditions within the chamber are controlled until the cells have healed to confluence.

9. Use according to claim 8 wherein temperature, pH, growth of microorganisms, gas and ion concentration within the chamber are controlled until the cells have healed to confluence.

10. Use according to any one of the preceding claims, wherein the genetic material is used in combination with one or more compounds selected from antibiotics, anaesthetics, chemotherapeutic agents, immunomodulators, and growth factors.

11. Use in the manufacture of a medicament of exogenous genetic material in combination with a chamber for culturing in vivo cells at a site on a patient, which chamber is formed of a flexible, moisture and gas impermeable material, and comprises an opening securable at the periphery to the skin of the patient,
wherein the combination is used in the manufacture of a medicament for use in a method for genetically engineering cells *in vivo* in a patient by introducing the exogenous genetic material into cells to be engineered wherein the chamber is attached at a site on a patient where the cells are to be engineered, the chamber containing a fluid medium suitable for culturing cells and being open to the cells to be engineered.

## Patentansprüche

1. Verwendung eines exogenen genetischen Materials zur Herstellung eines Arzneimittels zur Verwendung in einem Verfahren zur gentechnischen Behandlung von Zellen in vivo in einem Patienten durch Einbringen des exogenen genetischen Materials in die gentechnisch zu behandelnden Zellen, wobei eine aus einem flexiblen, feuchtigkeits- und gasundurchlässigen Material gebildete Kammer an einer Stelle des Patienten angebracht wird, wo die Zellen gentechnisch behandelt werden sollen, und die Kammer gegenüber den gentechnisch zu behandelnden Zellen offen ist und ein fluides Medium enthält, das zur Kultivierung von Zellen geeignet ist.

2. Verwendung nach Anspruch 1, dadurch gekennzeichnet, daß die Zellen Keratinocyten sind.

3. Verwendung nach Anspruch 1 oder 2, dadurch gekennzeichnet, daß das genetische Material in Form eines viralen Vektors oder Plasmids vorliegt.

4. Verwendung nach einem der Ansprüche 1 bis 3, dadurch gekennzeichnet, daß die Zellen gentechnisch behandelt werden, um exogenes genetisches Material zu exprimieren.

5. Verwendung nach Anspruch 4, dadurch gekennzeichnet, daß die Zellen gentechnisch behandelt werden, um Proteine zu exprimieren, die ausgewählt sind aus Hormonen, Immunmodulatoren, Gerinnungsfaktoren und Wachstumsfaktoren.

6. Verwendung nach einem der Ansprüche 1 bis 3, dadurch gekennzeichnet, daß die Zellen gentechnisch behandelt werden, um keine Proteine zu exprimieren, die eine Immunreaktion gegen die Zellen hervorrufen.

7. Verwendung nach Anspruch 6, dadurch gekennzeichnet, daß die Zellen keine HLA-Antigene exprimieren.

8. Verwendung nach einem der vorhergehenden Ansprüche, dadurch gekennzeichnet, daß die Kulturbedingungen innerhalb der Kammer kontrolliert werden, bis die Zellen zusammengewachsen sind.

9. Verwendung nach Anspruch 8, dadurch gekennzeichnet, daß die Temperatur, der pH-Wert, das Wachstum des Mikroorganismen und die Gas- und Ionenkonzentration innerhalb der Kammer kontrolliert werden, bis die Zellen zusammengewachsen sind.

10. Verwendung nach einem der vorhergehenden Ansprüche, dadurch gekennzeichnet, daß das genetische Material in Kombination mit einer oder mehreren Verbindungen verwendet wird, die ausgewählt sind aus Antibiotika, Anästhetika, chemotherapeutischen Mitteln, Immunmodulatoren und Wachstumsfaktoren.

11. Verwendung eines exogenen genetischen Materials in Kombination mit einer Kammer zur in vivo-Kultivierung von Zellen an einer Stelle eines Patienten bei der Herstellung eines Arzneimittels, wobei die Kammer aus einem flexiblen, feuchtigkeits- und gasundurchlässigen Material ausgebildet ist, und eine Öffnung aufweist, die an der Außenseite der Haut des Patienten befestigbar ist,
wobei die Kombination zur Herstellung eines Arzneimittels zur Verwendung in einem Verfahren zur gentechnischen Behandlung von Zellen in vivo in einem Patienten verwendet wird, indem man das exogene genetische Material in gentechnisch zu behandelnde Zellen einbringt, wobei die Kammer an einer Stelle eines Patienten angebracht ist, wo sich die gentechnisch zu behandelnden Zellen befinden, und die Kammer ein fluides Medium enthält, das zur Kultivierung der Zellen geeignet ist und gegenüber den gentechnisch zu behandelnden Zellen offen ist.

## Revendications

1. Utilisation d'un matériel génétique exogène dans la fabrication d'un médicament destiné à être utilisé dans un procédé de manipulation génétique de cellules *in vivo* chez un patient, où l'on introduit le matériel génétique exogène dans les cellules à manipuler, dans laquelle utilisation une enceinte, constituée d'un matériau flexible et imperméable aux gaz et à l'humidité, est fixée sur un patient, à l'endroit où se trouvent les cellules à manipuler, et cette enceinte est ouverte aux cellules à manipuler et contient un milieu liquide approprié pour la culture de cellules.

2. Utilisation conforme à la revendication 1, dans laquelle les cellules sont des kératinocytes.

3. Utilisation conforme à la revendication 1 ou 2, dans laquelle le matériel génétique se présente sous la forme d'un plasmide ou d'un vecteur viral.

4. Utilisation conforme à l'une des revendications 1 à 3, dans laquelle les cellules sont manipulées en vue de l'expression d'un matériel génétique exogène.

5. Utilisation conforme à la revendication 4, dans laquelle les cellules sont manipulées de manière à produire des protéines choisies parmi les hormones, les immunomodulateurs, les facteurs de coagulation et les facteurs de croissance.

6. Utilisation conforme à l'une des revendications 1 à 3, dans laquelle les cellules sont manipulées de manière à ne pas produire des protéines déclenchant une réaction immunitaire contre ces cellules.

7. Utilisation conforme à la revendication 6, dans laquelle les cellules ne produisent pas d'antigènes HLA.

8. Utilisation conforme à l'une des revendications précédentes, dans laquelle on règle les conditions de culture qui règnent à l'intérieur de l'enceinte jusqu'à ce que les cellules aient cicatrisé jusqu'à confluence.

9. Utilisation conforme à la revendication 8, dans laquelle on règle la température, le pH et les concentrations d'ions et de gaz qui règnent à l'intérieur de l'enceinte et l'on y maîtrise la prolifération de microorganismes, jusqu'à ce que les cellules aient cicatrisé jusqu'à confluence.

10. Utilisation conforme à l'une des revendications précédentes, dans laquelle on utilise le matériel génétique en combinaison avec un ou plusieurs composés choisis parmi les antibiotiques, les anesthésiques, les agents de chimiothérapie, les immunomodulateurs et les facteurs de croissance.

11. Utilisation, dans la fabrication d'un médicament, d'un matériel génétique exogène en combinaison avec une enceinte permettant de cultiver des cellules *in vivo* en un certain endroit chez un patient, laquelle enceinte est constituée d'un matériau flexible et imperméable aux gaz et à l'humidité et comporte une ouverture dont la périphérie peut être fixée sur la peau du patient,
dans laquelle utilisation l'on emploie cette combinaison dans la fabrication d'un médicament destiné à être utilisé dans un procédé de manipulation génétique de cellules *in vivo* chez un patient, où l'on introduit le matériel génétique exogène dans les cellules à manipuler, l'enceinte étant fixée sur un patient, à l'endroit où se trouvent les cellules à manipuler, et l'enceinte étant ouverte aux cellules à manipuler et contenant un milieu liquide approprié pour la culture de cellules.
